# EUROPÄISCHE PATENTSCHRIFT

(11) **EP 0 838 454 B1**
(45) Veröffentlichungstag und Bekanntmachung des Hinweises auf die Patenterteilung: **09.01.2002**
(21) Anmeldenummer: 97118702.6
(22) Anmeldetag: 28.10.1997
(51) Int. Cl.: C07D 211/58, C07C 231/04

(54) **Verfahren zur Acetylierung sterisch gehinderter Diamine**
Process for acetylation of sterically hindered diamies
Procédé d'acétylation des diamines stériquement encombrées

(30) Priorität: 28.10.1996 DE 19644806
(43) Veröffentlichungstag der Anmeldung: 29.04.1998
(73) Patentinhaber: BASF AKTIENGESELLSCHAFT, 67056 Ludwigshafen (DE)
(72) Erfinder: Julius, Manfred, Dr., 67117 Limburgerhof (DE)
(74) Vertreter: Isenbruck, Günter, Dr.

(56) Entgegenhaltungen:
- DE-A- 2 349 962
- DE-A- 3 901 246
- US-A- 4 326 067
- B. SCHELLHORN: LIEBIGS ANNALEN DER CHEMIE, Bd. 417, 1918, Seiten 118-25, XP002049695
- E. MÜLLER (HRSG.): "Houben-Weyl: Methoden der Organischen Chemie, Band VII/4" 1968 , GEORG THIEME VERLAG , STUTTGART, DE XP002049696 * Seite 124 - Seite 128 *

## Beschreibung

Die Erfindung betrifft ein Verfahren zur Acetylierung von Diaminen mit einer sterisch gehinderten Aminfunktion und einer weiteren sterisch nicht gehinderten Aminfunktion, wobei die Acetylierung an der sterisch nicht gehinderten Aminfunktion erfolgt.

Es ist bekannt, Amide durch Umsetzung von Aminen mit Keten herzustellen [(Houben Weyl, Band VII/4, S. 124-6 (1968)]. Nachteiligerweise reagiert das Keten dabei wegen seiner hohen Reaktivität auch mit sterisch gehinderten sekundären Aminfunktionen, wie in der älteren Anmeldung DE-A 196 34 147 beschrieben.

Des weiteren ist bekannt, Amine durch Umsetzung mit Acetanhydrid bei gleichzeitiger Bildung einer äquimolaren Menge an Essigsäure als Nebenprodukt zu acetylieren. Insbesondere gilt dies für die Herstellung von 4-Acetamido-2,2,6,6-tetramethyl-piperidin, vgl. 1) C. Harries, Liebigs Ann. Chem. 417, S. 120-121 (1918); 2a) E.G. Rozantsev in H. Ulrich, "Free Nitroxyl Radicals", S. 231, Plenum, London (1979); 2b) E.G. Rozantsev et al., Izv. Akad. Nauk SSSR, Ser. Khim. 8, 1477-1479 (1966); = Bull. Acad. Sci USSR, Div. Chem. Sci. 15, 1422-1423 (1966); 3) N.R. Plessas et al., Carbohydr. Res. 89, S. 219 (1981); 4) EP-B 0 389 419, S. 20, Z. 45-55 (Ciba-Geigy, 1990).

Außerdem können acetylierte Aminogruppen auch durch Umsetzung mit Acetylchlorid unter Freisetzung eines Moläquivalents Chlorwasserstoff hergestellt werden (DE-A 39 01 246, DE-A 23 49 962).

Eine weitere Möglichkeit zur Acetylierung, insbesondere zur Acetylierung von Diaminen mit einer sterisch gehinderten Aminfunktion und einer weiteren sterisch nicht gehinderten Aminfunktion an der zuletzt genannten besteht in der Reduktion eines Nitroxyl-Radikals, wie etwa 4-Acetamido-2,2,6,6-tetramethyl-piperidin-1-oxyl mit Eisencarbonylen oder anderen Reduktionsmitteln, vgl. H. Alper, J. Org. Chem. 38, 1417-1418 (1973).

Nachteilig bei der vorstehend beschriebenen Umsetzung von Aminen mit Acetanhydrid zur Herstellung von Acetamiden ist, daß bei der Umsetzung mit Acetanhydrid ein Moläquivalent Essigsäure als Koppelprodukt auftritt. Dies bildet zunächst ein Salz in Form eines Ammoniumacetats. Deshalb muß bei dem bekannten Verfahren in einem weiteren Schritt durch Umsetzung des Salzes mit einer mindestens stöchiometrischen Menge an Base - z.B. durch Umsetzung mit wäßriger Natronlauge - das Amin freigesetzt werden.

Alternativ dazu kann die Essigsäure oder auch das meist im Überschuß eingesetzte Acetanhydrid abdestilliert werden.

Die weiteren vorstehend erwähnten Verfahren sind ebenfalls entweder aufwendig oder unter ökologischen Gesichtspunkten nicht unbedenklich.

Bei den bekannten Verfahren zu Acetylierung von Diaminen mit einer sterisch gehinderten Aminfunktion und einer weiteren sterisch nicht gehinderten Aminfunktion kommt als zusätzliches Problem hinzu, daß sie nicht ausreichend selektiv sind.

Aufgabe der Erfindung ist es daher, ein Verfahren zur Acetylierung von Diaminen mit einer sterisch gehinderten Aminfunktion und einer weiteren sterisch nicht gehinderten Aminfunktion bereitzustellen, wobei die Acetylierung an der sterisch nicht gehinderten Aminfunktion erfolgt.

Diese Aufgabe wird erfindungsgemäß dadurchgelöst, daß ein Verfahren zur Acetylierung von Diaminen mit einer sterisch gehinderten Aminfunktion mit mehr als einem in α-Stellung zur Aminfunktion verzweigten Substituenten und einer weiteren sterisch nicht gehinderten Aminfunktion erfolgt, bereitgestellt wird, das dadurch gekennzeichnet ist, daß die sterisch gehinderte Aminfunktion Bestandteil eines Heterocyclus ist und das Diamin mit Keten umgesetzt wird.

Der Begriff "sterisch gehinderte Aminfunktion" bezeichnet eine Aminfunktion mit mehr als einem in α-Stellung zur Aminfunktion verzweigten Substituenten. Die sterisch gehinderte Aminofunktion ist Teil eines heterocyclischen Ringsystems. In 4-Amino-2,26,6-tetramethylpiperidin wird die sterische Hinderung durch die Methylsubstitution in 2- und 6-Stellung bedingt. Demgegenüber ist die primäre Aminogruppe in 4-Stellung sterisch nicht gehindert.

Das erfindungsgemäße Verfahren erfolgt unter sehr milden Reaktionsbedingungen. Insbesondere liegt die Reaktionstemperatur im Bereich von -30°C bis 65°C. Desweiteren wird die Selektivität des erfindungsgemäßen Vefahrens durch Reaktion in einem apriotischen Lösungsmittel, insbesondere in Tetrahydrofuran gefördert.

Das Verfahren wird bevorzugt nicht-katalysiert durchgeführt, es können jedoch auch Katalysatoren wie z.B. Trialkylamine oder 4-Dimethylaminopyridin (DMAP) eingesetzt werden.

Als Lösungsmittel für die Reaktion kommen polare oder unpolare, inerte, aprotische, organische Lösungsmittel wie z.B. Kohlenwasserstoffe oder Ether in Betracht. Bevorzugt verwendet man Ether, insbesondere Tetrahydrofuran. Gegebenenfalls kann die Reaktion auch ohne Lösungsmittel durchgeführt werden. Das erfindungsgemäße Verfahren wird besonders bevorzugt bei der Acetylierung von Diaminen mit sterisch gehinderter Aminfunktion und einer weiteren sterisch nicht gehinderten Aminfunktion eingesetzt, bei denen die sterisch gehinderte Aminfunktion Bestandteil eines N-Heterocyclus ist. Insbesondere handelt es sich bei dem N-Heterocyclus um 2,2,6,6-Tetramethylpiperidin (TAD). TAD ist ein wichtiges Zwischenprodukt für die Synthese von Lichtschutzmitteln vom HALS-Typ (HALS: Hindered Amine Light Stabilizer). Bei der ganz besonders bevorzugten Umsetzung des TAD mit Keten wird mit einer Selektivität von über 95 % die sterisch nicht gehinderte Aminfunktion mit dem Keten acetyliert, obwohl bekannt ist, daß unter normalen Umständen das Keten wegen seiner großen Reaktivität mit sterisch gehinderten Aminfunktionen reagiert. Die Umsetzung von TAD mit Keten erfolgt erfindungsgemäß bevorzugt drucklos und/oder diskontinuierlich, die Reaktion kann jedoch sowohl in einem Autoklaven unter Druck als auch kontinuierlich ausgeführt werden.

Das erfindungsgemäße Verfahren wird nachfolgend durch ein Beispiel erläutert:

### Beispiel

### Synthese von 4-Acetamido-2,2,6,6-tetramethyl-piperidin

In einem 0,5 l Vierhalskolben wurde eine Lösung von 46,88 g (0,30 Mol) 4-Amino-2,2,6,6-tetramethyl-piperidin (TAD) (Reinheit nach GC: 98,8 F1.-%) in 250 ml trockenem Tetrahydrofuran (THF) unter N₂ bei 0°C vorgelegt.

In diese Lösung leitete man unter Rühren während 1,5 h einen Keten-Gasstrom von ca. 0,5 Mol/h ein, wobei die Temperatur der Reaktionsmischung bei +1 bis -2°C gehalten wurde (Kühlbad) und sich die Lösung von hellgelb nach braun verfärbte. Der TAD-Umsatz betrug 100 % bei einer Selektivität von 95,3 % (nach GC; Bedingungen: 30 m RTX-5-Amine; 50-250°C, 5°C/Min). Der Reaktionsaustrag wurde bei 100 bis 20 mbar und 50°C am Rotationsverdampfer eingeengt und der Rückstand aus ca. 70 g Ethanol umkristallisiert. Das Produkt wurde abgesaugt, mit ca. 100 ml Petrolether 30/75 gewaschen und bei 0,5 mbar und 22°C getrocknet.
Ausbeute: 36,2 g (61,1 %) hellgelbe Kristalle.
Reinheit nach GC: 99,2 %
Smp.: 119-120°C

Aus der Mutterlauge konnten weitere 11,4 g (19,0 %) des Produkts in einer Reinheit von 97,7 % (GC) kristallisiert werden.

Die Gesamtausbeute an isoliertem Produkt betrug 80,1 % (ber. 100%iges Produkt).
¹³C-NMR (62,9 MHz, CDCl₃):
δ = 23,32 (q; CH₃-CO), 28,23 (q; 2 · CH₃), 34,65 (q; 2 · CH₃), 42,39 (d; CH-N), 44,95 (t; 2·CH₂-CH), 51,27 (s; 2·C(CH₃)₂), 169,84 (s; C=O).
Massenspektrum (EI): M⁺ = 198.
IR-Spektrum: 1640 und 1560 cm⁻¹ (sekundäres Amid).

## Patentansprüche

1. Verfahren zur Acetylierung von Diaminen mit einer sterisch gehinderten Aminfunktion mit mehr als einem in α-Stellung zur Aminfunktion verzweigten Substituenten und einer weiteren sterisch nicht gehinderten Aminfunktion, wobei die Actylierung an der sterisch nicht gehinderten Aminfunktion erfolgt, **dadurch gekennzeichnet, daß** die sterisch gehinderte Aminfunktion Bestandteil eines Heterocyclus ist und das Verfahren den Schritt umfaßt, daß das Diamin mit Keten umgesetzt wird.

2. Verfahren nach Anspruch 1, **dadurch gekennzeichnet, daß** die Umsetzung bei einer Temperatur im Bereich von -30°C bis 65°C erfolgt.

3. Verfahren nach Anspruch 1 oder 2, **dadurch gekennzeichnet, daß** die Umsetzung in einem aprotischen Lösungsmittel, insbesondere Tetrahydrofuran erfolgt.

4. Verfahren nach einem der Ansprüche 1 bis 3, **dadurch gekennzeichnet, daß** das Diamin 4-Amino-2,2,6,6 -Tetramethyl-piperidin (TAD) ist.

## Claims

1. A process for acetylating diamines having a sterically hindered amino function having more than one substituent branched in the α position with respect to the amino function, and a further, sterically unhindered amino function where the acetylation takes place at the sterically unhindered amino function, wherein the sterically hindered amino function is part of a heterocycle and which process comprises reacting the diamine with ketene.

2. A process as claimed in claim 1, wherein the reaction is carried out at from -30°C to 65°C.

3. A process as claimed in claim 1 or 2, wherein the reaction is carried out in an aprotic solvent, in particular tetrahydrofuran.

4. A process as claimed in any of claims 1 to 3, wherein the diamine is 4-amino-2,2,6,6-tetramethylpiperidine (TAD).

## Revendications

1. Procédé pour acétyler des diamines ayant une fonction amine à empêchement stérique comportant plus d'un substituant ramifié en position α par rapport à la fonction amine, et une autre fonction amine sans empêchement stérique, l'acétylation ayant lieu sur la fonction amine sans empêchement stérique, **caractérisé en ce que** la fonction amine à empêchement stérique fait partie d'un composé hétérocyclique, le procédé comportant l'étape de réaction de la diamine avec un cétène.

2. Procédé selon la revendication 1, **caractérisé en ce que** la réaction a lieu à une température comprise entre -30 et 65°C.

3. Procédé selon la revendication 1 ou 2, **caractérisé en ce que** la réaction a lieu dans un solvant aprotique, en particulier le tétrahydrofuranne.

4. Procédé selon l'une des revendications 1 à 3, **caractérisé en ce que** la diamine est la 4-amino-2,2,6,6-tétraméthyl-pipéridine (TAD).
